# EUROPÄISCHE PATENTANMELDUNG

(11) **EP 2 139 064 A1**
(43) Veröffentlichungstag der Anmeldung: **30.12.2009**
(21) Anmeldenummer: 09160409.0
(22) Anmeldetag: 15.05.2009
(51) Int. Cl.: H01Q 1/24, H01Q 1/36, H01Q 7/00, H01Q 9/16, H01Q 9/30, H01Q 9/38, H01Q 21/24, A61B 5/00, A61N 1/372

(54) **Patientengerät mit einer Antennenanordnung mit Polarisationsdiversität**

(30) Priorität: 23.06.2008 DE 102008002587
(71) Anmelder: Biotronik CRM Patent AG, 6341 Baar (CH)
(72) Erfinder: Merlin, Julian, 10713 Berlin (DE)
(74) Vertreter: Lindner-Vogt, Karin L.

(57) **Zusammenfassung**

Ein Patientengerät, welches eine Empfängeranordnung mit einer besonders hohen Empfangssensibilität besitzt. Das Patientengerät besitzt jeweils eine E-Feld-Antenne (11) und eine H-Feld-Antenne (21), wobei die H-Feld-Antenne als Rahmenantenne ausgeführt ist. Gemäß der Erfindung bildet eine durch die E-Feld-Antenne verlaufende Hauptachse einen Winkel von kleiner 30 Grad zu einer Normalen einer von der Rahmenantenne aufgespannten Fläche.

## Beschreibung

### Hintergrund der Erfindung

Die Erfindung betrifft ein Patientengerät mit einer Antennenanordnung mit Polarisationsdiversität.

Im medizintechnischen Bereich werden zunehmend sogenannte Patientengeräte verwendet, die vom Patienten mit sich geführt werden können und gewöhnlich in regelmäßigen Abständen über eine drahtlose Datenverbindung medizinische oder technische Daten von einem Implantat wie z.B. einem Herzschrittmacher eines Patienten empfangen und für eine ärztliche Kontrolle bereithalten oder über weitere Kommunikationsschnittstellen direkt zur medizinischen Überwachung an geeignete Einrichtungen weiter übermitteln. Da elektromedizinische Implantate nur über eine Operation ausgetauscht werden können und daher mehrere Jahre batteriegetrieben im Körper des Patienten verbleiben sollen, stellt die für eine solche Telemetriefunktion aufzuwendende zusätzliche Leistungsaufnahme einen kritischen Faktor bei der Umsetzung dar. Um die für eine zuverlässige Datenübertragung aufzuwendende Sendeleistung zu minimieren, ist eine besonders empfindliche Empfangsanordnung im Patientengerät erstrebenswert. Die vorliegende Erfindung hat daher zur Aufgabe, ein Patientengerät anzugeben, welches eine Empfängeranordnung mit einer besonders hohen Empfangssensibilität besitzt.

### Zusammenfassung der Erfindung

Um die oben angegebenen Anforderungen zu erfüllen, gibt die Erfindung ein Patientengerät an, welches über eine Sender/Empfängervorrichtung mit jeweils einer E-Feld-Antenne und einer H-Feld-Antenne verfügt, wobei die H-Feld-Antenne als Rahmenantenne ausgeführt ist. Gemäß der Erfindung bildet eine durch die E-Feld-Antenne verlaufende Hauptachse einen Winkel von kleiner 30 Grad zu einer Normalen einer von der Rahmenantenne aufgespannten Fläche.

Diese geometrische Anordnung besitzt den Vorteil, dass sich die E-Feld-Antenne und die H-Feld-Antenne nicht gegenseitig beeinflussen können. Unter "Hauptachse" sei in diesem Zusammenhang diejenige Achse gemeint, die sich eine größtmögliche Strecke innerhalb der E-Feld-Antenne befindet.

Die geringste Beeinflussung von E-Feld-Antenne und H-Feld-Antenne ergibt sich, wenn die Hauptachse wenigstens näherungsweise parallel oder besser genau parallel zu der Normalen ist.

Bei einer wegen ihrer besonders kompakten Bauform bevorzugten Ausführungsvariante verläuft die Hauptachse durch die aufgespannte Fläche. Besonders bevorzugt verläuft die Hauptachse durch einen Flächenschwerpunkt der aufgespannten Fläche.

Bevorzugt ist die E-Feld-Antenne und/oder die H-Feld-Antenne elektrisch verlängert. Dies kann im Fall der E-Feld-Antenne in bekannter Weise durch beispielsweise eine Spule am Speisepunkt der Antenne oder durch einen an der Spitze der E-Feld-Antenne befestigten Kondensator, im Fall der H-Feld-Antenne durch Vorsehen mehrerer Schleifenwindungen bewirkt werden.

Die E-Feld-Antenne kann als Stabantenne oder Wendelantenne ausgeführt sein.

Besonders bevorzugt weisen die E-Feld-Antenne und die H-Feld-Antenne eine Resonanzfrequenz zwischen 300 MHz und 500 MHz auf. Dieser Frequenzbereich ist besonders geeignet für die Übertragung von Daten aus medizintechnischen Implantaten.

Eine besonders bevorzugte Ausführungsform des erfindungsgemäßen Patientengerätes besitzt eine Antennenanordnung mit einem mechanisch besonders robusten Aufbau. Hierbei ist die E-Feld-Antenne als Viertelwellendipol mit einem Antennengegengewicht ausgeführt. Als "Gegengewicht" bezeichnet man in der Antennentechnik eine mit Masse verbundene metallische Vorrichtung, welche als Platte oder Stern ausgeführt sein kann und den Viertelwellendipol spiegelt, so dass dessen Abstrahlungsverhalten wenigstens näherungsweise dem eines Halbwellendipols entspricht. In der bevorzugten Ausführungsform dient das Antennengegengewicht gleichzeitig dazu, die als Rahmenantenne ausgeführte H-Feld-Antenne mechanisch zu stabilisieren, indem es (elektrisch isoliert) mit dieser an wenigstens einem Punkt der H-Feld-Antenne verbunden ist.

Besonders bevorzugt ist die Sender/Empfängervorrichtung des Patientengerätes ausgebildet, im Betrieb jeweils eine der E-Feld-Antenne und der H-Feld-Antenne abhängig von einer Empfangsstärke eines empfangenen Signals für den Empfang auszuwählen. Alternativ ist es aber auch möglich, die Signale beider Antennen zu summieren.

Wenigstens eine der E-Feld-Antenne und der H-Feld-Antenne kann ein von einem Kontroller abstimmbares Anpassungsnetzwerk aufweisen. Dies ermöglicht es, die Empfangs- bzw. Sendeleistung zu maximieren, indem im Betrieb die Anpassung der E-Feld-Antenne und/oder der H-Feld-Antenne entsprechend der jeweiligen durch die Umgebung gegebenen Randbedingungen korrigiert werden kann.

Das abstimmbare Anpassungsnetzwerk kann über eine Mehrzahl von parallelschaltbaren Kondensatoren verfügen, wobei der Kontroller ausgebildet ist, eine (zwischen keinem oder allen liegende) Teilmenge der Kondensatoren mit der wenigstens einen der E-Feld-Antenne und der H-Feld-Antenne zu verbinden und die verbleibenden Kondensatoren elektrisch von der wenigstens einen der E-Feld-Antenne und der H-Feld-Antenne zu isolieren.

Alternativ oder zusätzlich z.B. als "Fine-tuning" kann das abstimmbare Anpassungsnetzwerk eine Varaktordiode enthalten. Der Kontroller ist ausgebildet, über eine Induktivität eine Sperrspannung der Varaktordiode vorzugeben. Durch die Sperrspannung kann die Kapazität der Varaktordiode stufenlos eingestellt werden. Die Induktivität dient dazu, das hochfrequente Antennensignal von der Steuerspannung des Kontrollers zu entkoppeln.
Die E-Feld-Antenne und/oder die H-Feld-Antenne können über einen Richtkoppler an die Sender/Empfängervorrichtung angeschlossen sein. Der Richtkoppler ist ausgebildet, ein Messsignal an den Kontroller auszugeben. Das Messsignal gibt dabei ein Maß für von der der E-Feld-Antenne und/oder der H-Feld-Antenne reflektierter oder empfangener Leistung an. Das Messsignal ermöglicht vorteilhaft den Aufbau einer Regelschleife zur Einstellung des abstimmbaren Anpassungsnetzwerks.

Kurzbeschreibung der Abbildungen

### Es zeigen:

- Fig. 1: die Antennenanordnung eines ersten Ausführungsbeispiels des erfindungsgemäßen Patientengeräts;
- Fig. 2: die Antennenanordnung eines zweiten Ausführungsbeispiels des erfindungsgemäßen Patientengeräts;
- Fig. 3: in zwei Unterabbildungen Beispiele, wie die Antennenanordnungen mit einer als Platine ausgeführten elektronischen Schaltung des erfindungsgemäßen Patientengeräts mechanisch kombiniert werden kann;
- Fig. 4: eine erste Variante eines Anpassungsnetzwerks;
- Fig. 5: eine zweite Variante eines Anpassungsnetzwerks; und
- Fig. 6: ein Blockdiagramm einer Sender/Empfängervorrichtung mit Anpassungsnetzwerk.

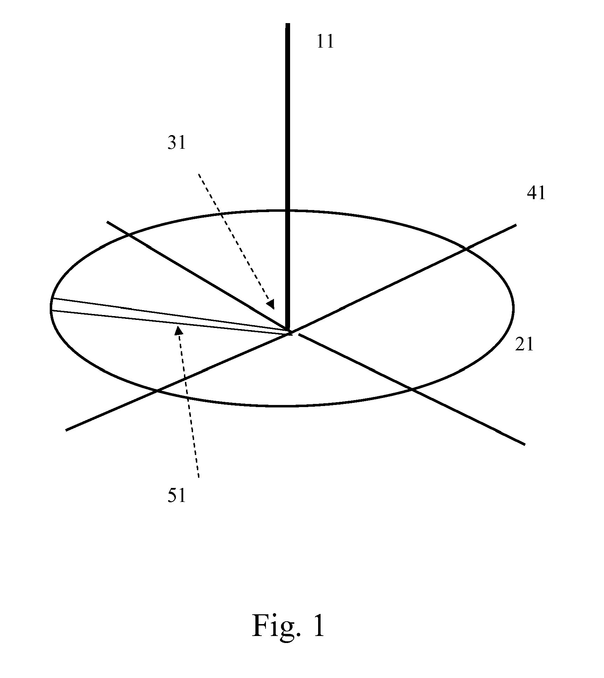

Fig. 1 zeigt die Antennenanordnung eines ersten Ausführungsbeispiels des erfindungsgemäßen Patientengeräts. Eine E-Feld-Antenne 11, welche vorzugsweise als elektrisch verlängerte Viertelwellen- oder Wendelantenne ausgeführt ist, ist im Zentrum einer kreisförmigen Rahmenantenne 21 so angeordnet, dass der Speisepunkt 31 der E-Feld-Antenne im Zentrum der Rahmenantenne 21 positioniert ist. Die Rahmenantenne 21 kann generell auch als Schleifenantenne ausgeführt sein, welche dann zwei oder mehr Wendeln aufweist. Die Rahmenantenne 21 kann selbstverständlich auch andere geometrische Formen annehmen wie etwa rechteckig, quadratisch, dreieckig oder sechseckig. Ein Antennengegengewicht 41 ist als rechtwinkliges Kreuz zweier starrer metallischer Leiter ausgebildet, dessen Kreuzungspunkt sich möglichst nahe am Speisepunkt 31 der E-Feld-Antenne 11 auf der von der E-Feld-Antenne 11 abgewandten Seite befindet. Die metallischen Leiter sind elektrisch mit der Massenzuleitung zum Speisepunkt 31 verbunden und derartig bemessen, dass sie die Rahmenantenne 21 mechanisch stützen, von welcher sie elektrisch isoliert sind. Das Antennengegengewicht 41 kann beispielsweise auch als Stern von metallischen Leitern oder als durchgehende Fläche ausgeführt sein. Die Rahmenantenne 21 wird über eine Speiseleitung 51 versorgt, welche eine massenumschirmte Signalleitung enthält. Vorzugsweise ist die Rahmenantenne 21 derartig flach aufgebaut, dass die von ihr umfasste Fläche wenigstens näherungsweise in einer Ebene liegt. Die Rahmenantenne 21 ist relativ zur E-Feld-Antenne 11 so angeordnet, dass eine Hauptachse der E-Feld-Antenne wenigstens näherungsweise parallel zu einer Flächennormalen der von der Rahmenantenne 21 umfassten Fläche ist. Aufgrund dieser Anordnung wird erreicht, dass sich die beiden Antennen gegenseitig nicht negativ beeinflussen. "Wenigstens näherungsweise parallel" bedeutet in diesem Zusammenhang einen Winkel von kleiner 30 Grad, besser jedoch von kleiner 10 Grad. Am besten ist der durch die geometrische Anordnung angestrebte Effekt, wenn die Hauptachse der E-Feld-Antenne innerhalb der üblichen Fertigungstoleranzen möglichst parallel zu der Normalen der von der H-Feld-Antenne umfassten Fläche ausgerichtet ist.

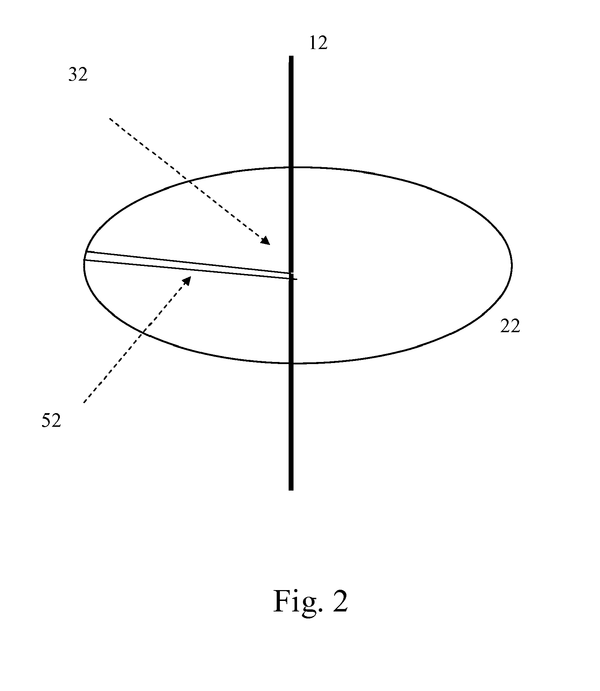

Fig. 2 zeigt die Antennenanordnung eines zweiten Ausführungsbeispiels des erfindungsgemäßen Patientengeräts. Bei diesem Ausführungsbeispiel ist die E-Feld-Antenne 12 als Halbwellendipol ausgeführt, weshalb sich der Speisepunkt 32 in der Mitte der E-Feld-Antenne 12 befindet. Als Halbwellendipol verfügt die E-Feld-Antenne 12 über kein Antennengegengewicht. Eine mechanische Verbindung der E-Feld-Antenne 12 mit der Rahmenantenne 22 wird daher vorzugsweise durch nichtleitende Materialien vorgenommen. Die Rahmenantenne 22 ist wiederum kreisförmig ausgebildet. Die E-Feld-Antenne 12 ist relativ zur Rahmenantenne 22 so angeordnet, dass der Speisepunkt 32 wenigstens näherungsweise im Mittelpunkt der von der Rahmenantenne 22 umfassten Fläche zu liegen kommt. Die Hauptachse der E-Feld-Antenne 22 ist wiederum wenigstens näherungsweise parallel zu einer Normalen der von der Rahmenantenne 22 umfassten Fläche. Die obigen Aussagen zu alternativen Formen der Rahmenantenne 22 gelten auch für dieses Ausführungsbeispiel. Die Speiseleitung 52 verbindet den Speisepunkt 32 der E-Feld-Antenne 12 mit der Rahmenantenne 22.

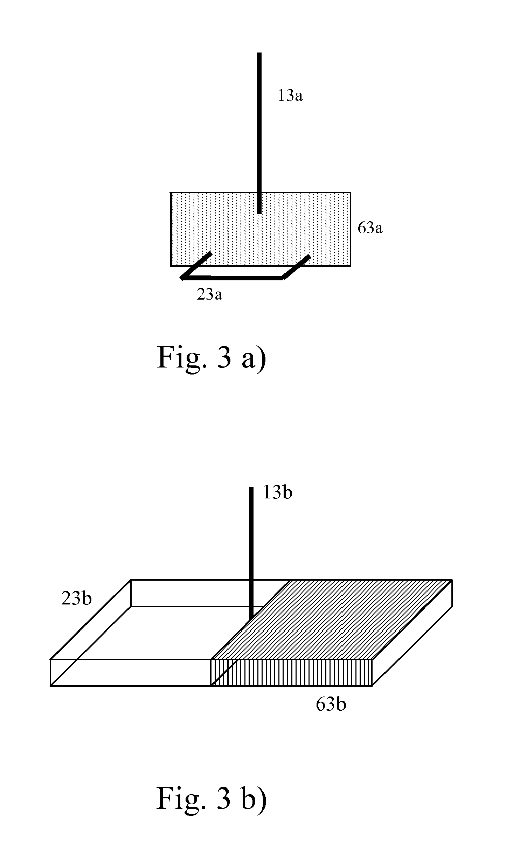

Fig. 3 zeigt in zwei Unterabbildungen Beispiele, wie die Antennenanordnungen mit einer als Platine ausgeführten elektronischen Schaltung des erfindungsgemäßen Patientengeräts mechanisch kombiniert werden kann. In der ersten Teilabbildung ist eine E-Feld-Antenne 13a mittig an einem Rand einer Platine 63a, auf welcher die elektronische Schaltung der Sender/Empfängervorrichtung sowie etwaige weitere Komponenten wie Anpassungsnetzwerke und dergleichen aufgebracht sind, befestigt, so dass die Hauptachse der E-Feld-Antenne 13a wenigstens näherungsweise parallel zu einer von der Platine 63a beschriebenen Ebene ausgerichtet ist. Eine Rahmenantenne 23a ist im unteren Bereich der Platine 63a senkrecht zu deren beider Oberflächen befestigt. Die Rahmenantenne 23a ist aus einem steifen leitenden Material gefertigt, so dass eine zusätzliche mechanische Stabilisierung nicht vonnöten ist. Die Speisepunkte sowohl der E-Feld-Antenne 13a als auch der Rahmenantenne 23a liegen auf der Platine, so dass eine besonders einfache Verbindung mit den elektronischen Schaltkreisen der Sender/Empfängervorrichtung möglich ist. Wie schon zuvor, ist die Hauptachse der E-Feld-Antenne 13a wenigstens näherungsweise parallel zu einer Normalen einer von der Rahmenantenne 23a umfassten Fläche ausgerichtet.

Im Ausführungsbeispiel der zweiten Teilabbildung ist die E-Feld-Antenne 13b senkrecht zur Platine 63b angeordnet. Die Rahmenantenne 23b liegt in einer Ebene mit der Platine 63b und umfasst diese in einer Hälfte der Rahmenantenne 23b. Auch hier ist die E-Feld-Antenne 13b wiederum wenigstens näherungsweise parallel zu einer Flächennormalen der von der Rahmenantenne 23b umfassten Fläche angeordnet. Die Speisepunkte beider Antennen liegen wiederum auf der Platine 63b.

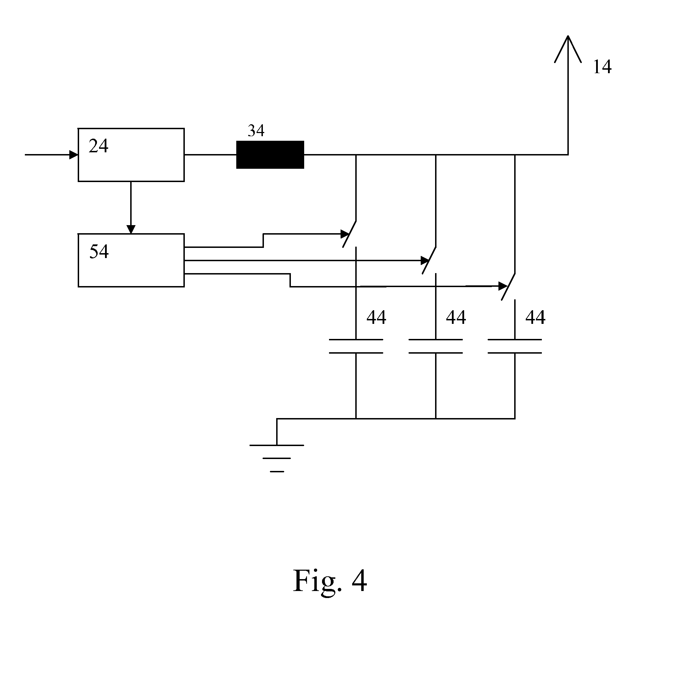

Fig. 4 zeigt eine erste Variante eines Anpassungsnetzwerks. Das Schaltungsbeispiel zeigt nur ein Anpassungsnetzwerk für eine E-Feld-Antenne 14, welche durch die zwischen die E-Feld-Antenne 14 und den Richtkoppler 24 geschaltete Induktivität 34 elektrisch verlängert wird. An die Sender/Empfängervorrichtung, an welche die E-Feld-Antenne 14 über den Richtkoppler 24 angeschlossen ist, können weitere Antennen wie insbesondere eine H-Feld-Antenne angeschlossen sein. Um die Anpassung der E-Feld-Antenne 14 veränderlich zu gestalten und somit die E-Feld-Antenne 14 an veränderliche Abstrahlungs- bzw. Empfangsbedingungen anpassen zu können, sind im Beispiel drei Kondensatoren 44 zwischen die Antennenleitung und Masse schaltbar angeordnet. Die Kondensatoren 44 sind dabei über Schalter, vorzugsweise Transistoren, mit der Antennenleitung verbindbar ausgeführt. Ein Kontroller 54 empfängt vom Richtkoppler 24 ein Signal vom Richtkoppler 24, welches insbesondere im Sendefall ein Maß für die Anpassung der Antenne darstellt. Der Kontroller 54 ist bestrebt, das vom Richtkoppler 24 empfangene (Leistungs)Signal zu minimieren oder unter ein vorbestimmtes Maß zu senken, indem er die Antennenanpassung dynamisch über geeignete Schaltsignale für die an den Kondensatoren 44 angeordneten Schalter verändert. Natürlich ist das Ausführungsbeispiel nicht auf eine Anzahl von drei Kondensatoren 44 beschränkt. Vorteilhafterweise weisen die Kondensatoren 44 binär steigende Kapazitätswerte auf, so dass sich aus einer minimalen Anzahl Kondensatoren 44 eine größtmögliche Zahl verschiedener Kapazitätswerte kombinieren lässt.

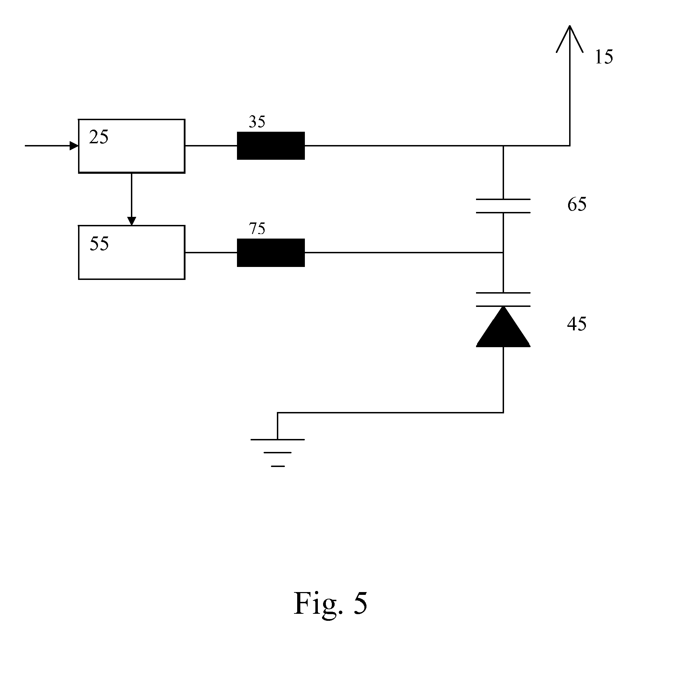

Fig. 5 zeigt eine zweite Variante eines Anpassungsnetzwerks. Das zweite Ausführungsbeispiel unterscheidet sich vom ersten durch die Art, in der die variable Kapazität zur Anpassung realisiert ist. Prinzipiell ist es auch möglich, beide Varianten miteinander zu kombinieren. Das Anpassungsnetzwerk der zweiten Variante umfasst eine Varaktordiode 45, welche in Sperrrichtung zwischen Masse und einen Kondensator 65 geschaltet ist. Ein zweiter Anschluss des Kondensators 65 ist an die Antennenleitung angeschlossen. Die Varaktordiode 45 weist eine variable Kapazität auf, welche von der Höhe der Sperrspannung über die Varaktordiode 45 abhängt. Die Sperrspannung und damit die Anpassung der Antenne wird vom Kontroller 55 in Abhängigkeit von dem mit dem Richtkoppler 25 bereitgestellten Signal, welches ein Maß für die Anpassung der Antenne darstellt, eingestellt. Die veränderliche Sperrspannung bewirkt eine Änderung der Kapazität der Varaktordiode. Die zweite Induktivität 75 dient dazu, das hochfrequente Sende- bzw. Empfangssignal von der im Kontroller angeordneten Gleichspannungsquelle, welche die Sperrspannung erzeugt, zu entkoppeln.

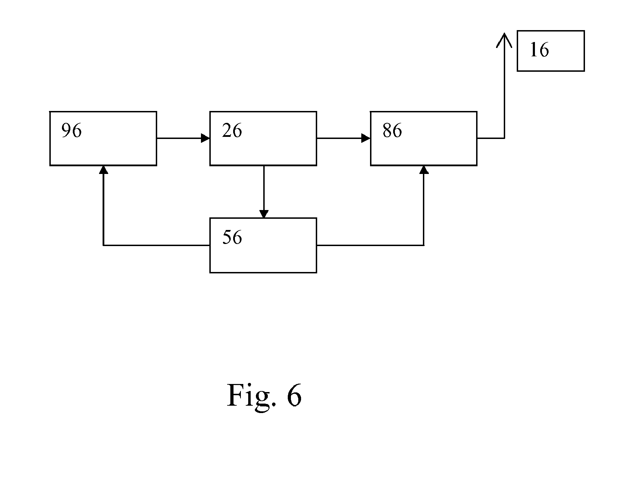

Fig. 6 zeigt ein Blockdiagramm einer Sender/Empfängervorrichtung mit Anpassungsnetzwerk. Die Sender/Empfängervorrichtung umfasst einen RF-Schaltkreis 96, welcher unter anderem einen Leistungsverstärker zur Erzeugung des Sendesignals enthält. Die Sender/Empfängervorrichtung ist vorzugsweise im Halbduplex-Betrieb umschaltbar zwischen einem Sende- und einem Empfangsmodus. Dementsprechend umfasst der RF-Schaltkreis 96 auch für den Empfang benötigte Schaltungskomponenten wie einen rauscharmen Verstärker und dergleichen. Das Sendesignal des RF-Schaltkreises 96 wird über einen Messschaltkreis 26, welcher vorzugsweise als Richtkoppler ausgeführt ist, an das Anpassungsnetzwerk 86 gegeben. Gleichzeitig erzeugt der Messschaltkreis 26 ein Messsignal für den Kontroller 56, welcher anhand des Messsignals bestimmt, wie gut die an das Anpassungsnetzwerk 86 angeschlossene Antenne 16 angepasst ist. Der Kontroller 56 erzeugt geeignete Steuersignale für den RF-Schaltkreis 96 und das Anpassungsnetzwerk 86, um die Anpassung der Antenne derart zu verändern, dass die Anpassung optimiert oder auf ein bestimmtes Maß gebracht wird.

## Patentansprüche

1. Patientengerät mit einer Sender/Empfängervorrichtung mit jeweils einer E-Feld-Antenne und einer H-Feld-Antenne, wobei die H-Feld-Antenne als Rahmenantenne ausgeführt ist, **gekennzeichnet dadurch, dass** eine durch die E-Feld-Antenne verlaufende Hauptachse einen Winkel von kleiner 30 Grad zu einer Normalen einer von der Rahmenantenne aufgespannten Fläche bildet.

2. Das Patientengerät nach Anspruch 1, bei der die Hauptachse parallel zu der Normalen ist.

3. Das Patientengerät nach einem der Ansprüche 1 oder 2, bei der die Hauptachse durch die aufgespannte Fläche verläuft.

4. Das Patientengerät nach Anspruch 3, bei dem die Hauptachse durch einen Flächenschwerpunkt der aufgespannten Fläche verläuft.

5. Das Patientengerät nach einem der vorhergehenden Ansprüche, bei der die E-Feld-Antenne elektrisch verlängert ist.

6. Das Patientengerät nach einem der vorhergehenden Ansprüche, bei der die H-Feld-Antenne elektrisch verlängert ist.

7. Das Patientengerät nach einem der vorhergehenden Ansprüche, bei der die E-Feld-Antenne als Stabantenne oder Wendelantenne ausgeführt ist.

8. Das Patientengerät nach einem der vorhergehenden Ansprüche, wobei die E-Feld-Antenne und die H-Feld-Antenne eine Resonanzfrequenz zwischen 300 MHz und 500 MHz aufweisen bzw. im Bereich zwischen 300 MHz und 500 MHz betrieben werden.

9. Das Patientengerät nach einem der vorhergehenden Ansprüche, wobei die E-Feld-Antenne als Viertelwellendipol mit einem Antennengegengewicht ausgeführt ist und das Antennengegengewicht die H-Feld-Antenne mechanisch stabilisiert.

10. Das Patientengerät nach einem der vorhergehenden Ansprüche, wobei die Sender/Empfängervorrichtung des Patientengeräts ausgebildet ist, im Betrieb jeweils eine der E-Feld-Antenne und der H-Feld-Antenne abhängig von einer Empfangsstärke eines empfangenen Signals für den Empfang auszuwählen.

11. Das Patientengerät nach einem der vorhergehenden Ansprüche, bei der wenigstens eine der E-Feld-Antenne und der H-Feld-Antenne ein von einem Kontroller abstimmbares Anpassungsnetzwerk aufweist.

12. Das Patientengerät von Anspruch 11, bei dem das abstimmbare Anpassungsnetzwerk über eine Mehrzahl von parallelschaltbaren Kondensatoren verfügt, wobei der Kontroller ausgebildet ist, eine Teilmenge der Kondensatoren mit der wenigstens einen der E-Feld-Antenne und der H-Feld-Antenne zu verbinden und die verbleibenden Kondensatoren elektrisch von der wenigstens einen der E-Feld-Antenne und der H-Feld-Antenne zu isolieren.

13. Das Patientengerät nach einem der Ansprüche 11 oder 12, bei dem das abstimmbare Anpassungsnetzwerk eine Varaktordiode enthält und bei dem der Kontroller ausgebildet ist, über eine Induktivität eine Sperrspannung der Varaktordiode vorzugeben.

14. Das Patientengerät nach einem der Ansprüche 11 bis 13, bei dem die wenigstens eine der E-Feld-Antenne und der H-Feld-Antenne über einen Richtkoppler an die Sender/Empfängervorrichtung angeschlossen ist, wobei der Richtkoppler ausgebildet ist, ein Messsignal an den Kontroller auszugeben, und das Messsignal ein Maß für von der wenigstens einen der E-Feld-Antenne und der H-Feld-Antenne reflektierter oder empfangener Leistung angibt.
